# EUROPÄISCHE PATENTSCHRIFT

(11) **EP 2 803 383 B1**
(45) Veröffentlichungstag und Bekanntmachung des Hinweises auf die Patenterteilung: **09.03.2016**
(21) Anmeldenummer: 14158920.0
(22) Anmeldetag: 11.03.2014
(51) Int. Cl.: A61N 1/362, A61N 1/365

(54) **Anordnung mit einem externen Herzschrittmacher**
Assembly with an external pacemaker
Dispositif doté d'un stimulateur cardiaque externe

(30) Priorität: 15.05.2013 DE 102013008318
(43) Veröffentlichungstag der Anmeldung: 19.11.2014
(73) Patentinhaber: Osypka AG, 79618 Rheinfelden-Herten (DE)
(72) Erfinder: Bolz, Armin, 90154 Buckenhof (DE); Paetow, Frank, 91080 Uttenreuth (DE); Seufert, Manuel, 91054 Erlangen (DE); Schwarz, Simon, 90765 Fürth (DE); Tröger, Tobias, 91052 Erlangen (DE); Braun, Michael, 89231 Neu-Ulm (DE); Göttsche, Thorsten, 79618 Rheinfelden-Herten (DE); Lewalter, Thorsten, 80802 München (DE); Lösch, Thomas, 41179 Mönchengladbach (DE)
(74) Vertreter: Herrmann, Uwe

(56) Entgegenhaltungen:
- US-A- 4 705 043
- US-A1- 2002 151 884
- US-A1- 2003 233 129
- US-A1- 2006 173 498
- US-A1- 2007 255 150

## Beschreibung

Die vorliegende Erfindung betrifft eine Anordnung mit einem externen Herzschrittmacher.

Im Rahmen von herzchirurgischen Eingriffen werden häufig externe, d. h. nicht implantierte Herzschrittmacher zur temporären kardialen Stimulation und intrakardialen EKG-Überwachung eingesetzt. Diese Stimulation und die Überwachung erfolgen mit Hilfe von Elektroden. Diese sind beispielsweise auf dem Herzmuskel aufgenäht und über Drähte mit dem externen Herzschrittmacher verbunden.

Nach herzchirurgischen Eingriffen kann es zu Herzrhythmusstörungen, wie beispielsweise dem sogenannten "Vorhofflimmern" kommen. Um derartigen Herzrhythmusstörungen entgegenzuwirken, ist das Verfahren der sogenannten Kardioversion bekannt, bei der ein Stromstoß/Schock getriggert auf die Herzspannungskurve (EKG) an das Herz abgegeben wird.

Sobald eine R-Zacke in der Herzspannungskurve erkannt wird, wird der Schock ausgelöst. Durch diesen Stromstoß wird die unkontrollierte Erregungsbildung unterbrochen und die Aktivität der Herzmuskelzellen wird auf einen Sinusrhythmus synchronisiert.

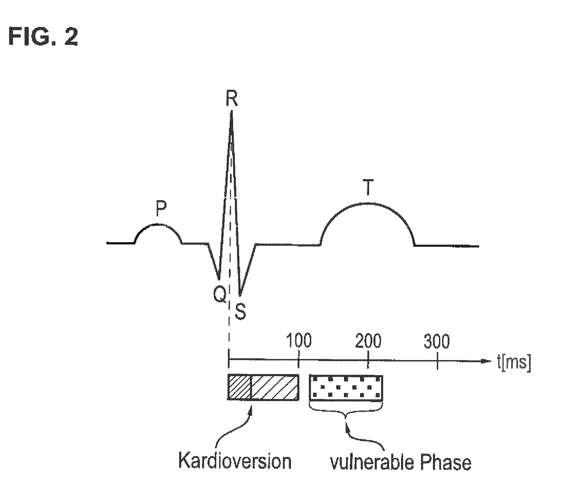

Figur 2 zeigt in exemplarischer Darstellung den Zeitpunkt der Kardioversion im EKG.

Aus Figur 2 ist ersichtlich, dass der Stromstoß getriggert durch das EKG bzw. die R-Zacke abgegeben wird. Dies erfolgt in dem hier dargestellten Ausführungsbeispiel zu dem Zeitpunkt, der durch den Pfeil oberhalb des Begriffes "Kardioversion" dargestellt ist. Der Impuls wird beispielsweise ca. 20 ms nach der R-Zacke appliziert, wie dies in Figur 2 angedeutet ist.

Grundsätzlich sind mehrere Möglichkeiten denkbar, wie die Triggerung des Kardioversionsschocks erfolgen kann. Denkbar ist es beispielsweise, die Herzspannungskurve als Oberflächen-EKG-Signal durch einen EKG-Monitor aufzuzeichnen und auszuwerten (R-Zackenerkennung). In diesem Fall wird nur das Triggersignal über eine Datenverbindung an den externen Herzschrittmacher weitergegeben. Auch ist es möglich, die Herzspannungskurve als Oberflächen-EKG-Signal durch einen EKG-Monitor aufzuzeichnen. Dieses Signal wird über eine Datenverbindung an den externen Herzschrittmacher weitergegeben und dort auf R-Zacken hin ausgewertet. Eine weitere Möglichkeit besteht darin, dass der externe Herzschrittmacher selbsttätig über Elektroden bzw. Herzdrähte die Herzspannungskurve aufzeichnet und die R-Zackenerkennung durchführt.

Wie dies aus der schematischen Darstellung gemäß Figur 2 hervorgeht, ist die korrekte Erkennung der R-Zacken von besonderer Bedeutung, da bei einer fehlerhaften Detektion der Kardioversionsschock möglicherweise innerhalb der ebenfalls dargestellten vulnerablen Phase des Herzens abgegeben wird. Dies kann im ungünstigsten Fall zu Kammerflimmern und zum Tode des Patienten führen.

Der vorliegenden Erfindung liegt daher die Aufgabe zugrunde, eine Anordnung der eingangs genannten Art dahingehend weiterzubilden, dass eine korrekte Abgabe des Kardioversionsschocks gewährleistet ist.

Diese Aufgabe wird durch eine Anordnung mit den Merkmalen des Anspruchs 1 gelöst.

Es ist vorgesehen, dass die Anordnung außer dem externen Herzschrittmacher eine Ausgabeeinrichtung, insbesondere eine Anzeigeeinrichtung aufweist, wobei der externe Herzschrittmacher erste Mittel zur Erzeugung von Impulsen zur regelmäßigen Stimulation des Herzmuskels im VVT-Modus und zweite Mittel zur Erzeugung eines Stromstoßes bzw. Kardioversionsschocks zum Zwecke der Kardioversion aufweist. Des Weiteren sind Aufzeichnungsmittel zur Ermittlung der Herzspannungskurve oder eines oder mehrerer Abschnitte der Herzspannungskurve vorgesehen sowie dritte Mittel, die mit der Ausgabeeinrichtung in Verbindung stehen und die so ausgebildet sind, dass sie der Ausgabeeinrichtung ein oder mehrere Signale zuführen, die sowohl die Herzspannungskurve bzw. den wenigstens einen Abschnitt der Herzspannungskurve sowie im VVT-Modus seitens des Herzschrittmachers erzeugte Impulse umfassen.

Erfindungsgemäß ist somit vorgesehen, dass der Ausgabeeinrichtung, wie beispielsweise einem Display ein Signal zugeführt wird, das sowohl die Herzspannungskurve (EKG) sowie auch die vom Herzschrittmacher erzeugten Impulse umfasst. Dieses Signal wird dann der Ausgabeeinrichtung zugeführt.

Der Begriff "Herzspannungskurve" umfasst im Rahmen der vorliegenden Erfindung sowohl eine vollständige (durchgehende) Herzspannungskurve, als auch eine oder mehrere Abschnitte von dieser, wie beispielsweise die R-Zacken.

Der Begriff "Aufzeichnungsmittel" umfasst Mittel, die geeignet sind, die Herzspannungskurve zu ermitteln. Diese können mit einem Speicher zum Speichern der Herzspannungskurve sowie auch ohne Speicher ausgeführt sein.

Handelt es sich bei der Ausgabeeinrichtung um eine Anzeige, wie z.B. ein Display ermöglicht dies die visuelle Überprüfung der R-Zackenerkennung eines externen Herzschrittmachers, indem das Herz durch die ersten Mittel im VVT-Modus stimuliert wird. Die abgegebenen Stimulationsimpulse können beispielsweise auf einem EKG-Monitor, der über eine Schrittmachererkennung verfügt, optisch dargestellt werden.

Auch andere Möglichkeiten der Anzeige, wie z.B. eine akustische Anzeige sind möglich und von der Erfindung umfasst.

Auf diese Weise ist es möglich, durch einen Nutzer der Anordnung erkennen zu können, ob der externe Herzschrittmacher zum gewünschten Zeitpunkt bzw. ob dieser überhaupt die Stimulationsimpulse abgibt. Falls dies nicht der Fall ist, kann darauf rückgeschlossen werden, dass die R-Zackenerkennung oder eine sonstige Funktionalität des externen Herzschrittmachers fehlerhaft ist. Der Anwender hat dann die Möglichkeit, den Kardioversionsvorgang aus Sicherheitsgründen abzubrechen und alternativ beispielsweise einen externen Defibrillator zur Therapie zu verwenden.

Durch die korrekte Abgabe des Stimulationssignals nach Erkennen der R-Zacke kann hingegen auf eine ordnungsgemäße Funktion des externen Herzschrittmachers geschlossen werden, so dass dieser selbst zur Therapie verwendet werden kann.

Die dritten Mittel können wie auch die anderen Mittel durch ein einziges oder durch mehrere Elemente gebildet werden. Die dritten Mittel können beispielsweise durch die EKG-Aufzeichnungsmittel zur Ermittlung der Herzspannungskurve gebildet werden oder mit diesen in Verbindung stehen. Des Weiteren können die dritten Mittel eine Einheit umfassen, die die Abgabe eines Stimulationsimpulses durch den Herzschrittmacher erfassen.

Der VVT-Modus ist dadurch gekennzeichnet, dass bei einer wahrgenommenen ventrikulären Herzeigenaktivität ein Stimulationsimpuls im Ventrikel (Triggerung) abgegeben wird. Die Abkürzung "VVT" kennzeichnet mit dem ersten "V" den Stimulationsort (Ventrikel), mit dem zweiten "V" den Registrierungsort (Ventrikel) und mit dem dritten Buchstaben, d. h. dem "T" die Betriebsart (getriggert). Getriggerte Herzschrittmacher zeichnen sich dadurch aus, dass ein registriertes Herzsignal zur Impulsabgabe führt. Im Gegensatz dazu wird bei Herzschrittmachern im inhibierten Modus die Abgabe eines Impulses bei herzeigener Aktivität unterdrückt.

Im Falle des VVT-Modus wird im Falle der fehlenden Herzeigenaktivität im Ventrikel eine Stimulation mit einer vorgegebenen Rate durchgeführt.

Aufgrund der Tatsache, dass bei der Stimulation im VVT-Modus jeder bei Eigenaktivität des Ventrikels abgegebene Impuls eine vom Herzschrittmacher erkannte R-Zacke signalisiert, kann der Anwender die Pulsabgabe und damit auch die R-Zackenerkennung durch die Verwendung eines EKG-Monitors mit Schrittmachererkennung oder durch einen sonstigen Monitor überprüfen. EKG-Monitore erkennen die durch die ersten Mittel abgegebenen Stimulationsimpulse im Oberflächen-EKG und können diese auf dem Monitor markieren.

Das an die Ausgabeeinrichtung abgegebene Signal kann für einen Nutzer wahrnehmbar sein und/oder in der Anordnung weiterverarbeitet werden, insbesondere für die Entscheidung, ob ein Kardioversionsimpuls abgegeben werden soll oder nicht.

Die Anordnung weist somit Auswertemittel auf, die den zeitlichen Zusammenhang zwischen der R-Zacke der Herzspannungskurve und den im VVT-Modus seitens des Herzschrittmachers erzeugten Impulsen ermitteln. Bei korrekter R-Zackenerkennung des externen Herzschrittmachers wird der durch die ersten Mittel abgegebene Stimulationsimpuls im zeitlichen Verlauf unmittelbar nach der R-Zacke des EKG-Signals angezeigt.

An dieser Stelle wird darauf hingewiesen, dass der Begriff "externer Herzschrittmacher" für einen Herzschrittmacher steht, der nicht implantiert ist. Von bzw. zu dem externen Herzschrittmacher verlaufen ein oder mehrere Drähte zum Herzen, die einerseits zur Abgabe der Stimulationsimpulse, zur Wahrnehmung der Herzeigenaktivität und andererseits zur Abgabe des Kardioversionsschocks dienen.

Wird der Stimulationsimpuls korrekt erzeugt, kann der Anwender auf eine korrekte Funktion des externen Herzschrittmachers und damit auch auf eine korrekte Anwendung der Kardioversion schließen.

Weiterhin kann vorgesehen sein, dass Warnmittel vorgesehen sind, die mit den Auswertemitteln in Verbindung stehen, wobei die Warnmittel derart ausgebildet sein können, dass sie ein Warnsignal abgeben, wenn der genannte zeitliche Zusammenhang zwischen der R-Zacke, der Herzspannungskurve und dem im VVT-Modus seitens des Herzschrittmachers erzeugten Impuls fehlerhaft ist. Dazu gehört auch der Fall, dass überhaupt kein Impuls nach vorliegender R-Zacke durch die ersten Mittel abgegeben wird.

Weiterhin kann vorgesehen sein, dass Sperrmittel vorgesehen sind, die mit den genannten Auswertemitteln in Verbindung stehen, wobei diese Sperrmittel derart ausgebildet sind, dass sie die Funktion der zweiten Mittel blockieren, wenn der genannte zeitliche Zusammenhang zwischen der R-Zacke der Herzspannungskurve und den im VVT-Modus seitens des Herzschrittmachers erzeugten Impulses fehlerhaft ist. In diesem Fall wird die Abgabe eines Stromstoßes durch die zweiten Mittel, d. h. eines Kardioversionsschocks unterbunden.

Dies kann beispielsweise dann der Fall sein, wenn nach dem Vorliegen einer R-Zacke kein Stimulationsimpuls festgestellt werden kann.

In weiterer Ausgestaltung der Erfindung ist vorgesehen, dass die Anordnung wenigstens ein Betätigungselement aufweist, das derart ausgebildet ist, dass es bei seiner Betätigung das erste Mittel zur Erzeugung von Impulsen zur regelmäßigen Stimulation des Herzmuskels im VVT-Modus aktiviert. Denkbar ist es beispielsweise, dass die Anordnung, insbesondere der Herzschrittmacher selbst eine "Schocktaste" oder dergleichen aufweist, die bei ihrer Betätigung den Herzschrittmacher von dem normalen Herzschrittmachermodus in den VVT-Modus bringt.

Weiterhin kann vorgesehen sein, dass die Anordnung ein Betätigungselement aufweist, das derart ausgebildet ist, dass es bei seiner Betätigung das Aufladen des Kondensators vornimmt.

In einer weiteren Ausgestaltung ist das Betätigungselement so ausgebildet, dass es bei seiner Betätigung über eine bestimmte Zeitspanne hinweg die zweiten Mittel zur Erzeugung eines Stromstoßes aktiviert. Wird somit das Betätigungselement lange genug betätigt, beispielsweise 15 Sekunden, ist der Ladevorgang für den Kondensator abgeschlossen und es erfolgt ein Kardioversionsschock, nachdem eine R-Zacke erkannt wurde.

In einer weiteren Ausgestaltung der Erfindung ist vorgesehen, dass das Betätigungselement so ausgebildet ist, dass es nach dem Loslassen vor Ablauf einer bestimmten Zeitspanne, beispielsweise 15 Sekunden, derart auf die zweiten Mittel einwirkt, dass kein Stromstoß abgegeben wird.

Stellt der Nutzer fest, dass der Herzschrittmacher nicht ordnungsgemäß arbeitet, beispielsweise weil der zeitliche Zusammenhang zwischen der R-Zacke und dem Stimulationsimpuls im VVT-Modus nicht korrekt ist oder weil kein Stimulationsimpuls abgegeben wird, kann der Nutzer durch Loslassen der Betätigungstaste die Kardioversion verhindern und das Aufladen des Kondensators abbrechen. Denkbar ist es, dass der Herzschrittmacher dann wieder in den vom Bediener eingestellten Pacer-Modus, d.h. in den Normalbetrieb des Herzschrittmachers übergeht.

In weiterer Ausgestaltung der Erfindung ist vorgesehen, dass es sich bei den vorgenannten Betätigungselementen um ein- und dasselbe Betätigungselement handelt, wie beispielsweise einen Druckknopf, ein Feld auf einem Touchscreen-Monitor, eine Taste etc.

In einer weiteren Ausgestaltung der Erfindung ist vorgesehen, dass die Ausgabeeinrichtung bzw. die Anzeigeeinrichtung Bestandteil des Herzschrittmachers ist oder dass die Anordnung einen EKG-Monitor umfasst und dass die Anzeigeeinrichtung Bestandteil des EKG-Monitors ist.

Bei dem EKG-Monitor kann es sich um einen solchen mit Herzschrittmachererkennung handeln. Auf dem Display des EKG-Monitors kann somit das EKG-Signal sowie auch die von dem Herzschrittmacher abgegebenen Impulse dargestellt werden.

Die Erfindung betrifft des Weiteren ein Verfahren zur Überprüfung der Funktionsfähigkeit eines Herzschrittmachers, wobei der Herzschrittmacher in einem normalen Betriebsmodus betreibbar ist, in dem er Stimulationsimpulse abgibt, und in einem VVT-Modus betreibbar ist, in dem er einen Stromstoß bzw. Kardioversionsschock zum Zwecke der Kardioversion abgibt, wobei das Verfahren die Ermittlung der Herzspannungskurve und die Ermittlung von seitens des Herzschrittmachers im VVT-Modus abgegebenen Stimulationsimpulsen und die Prüfung des zeitlichen Zusammenhangs zwischen dem Vorhandensein einer R-Zacke der Herzspannungskurve und einem im VVT-Modus abgegebenen Stimulationsimpuls und die Ausgabe eines auf der Prüfung basierenden Signals umfasst.

Das Signal kann für einen Nutzer wahrnehmbar sein, z.B. kann es sich um ein optisch und/oder akustisch wahrnehmbares Signal handeln. Es kann sich auch um ein nicht durch einen Nutzer wahrnehmbares Signal handeln, das z.B. dazu führt, dass der Kardioversionsschock abgegeben wird oder dessen Abgabe unterbunden wird.

Vorzugsweise ist vorgesehen, dass die Ausgabe des Signals mittels eines EKG-Monitors erfolgt, der über eine Schrittmachererkennung verfügt.

Die Erfindung betrifft des Weiteren die Verwendung eines externen Herzschrittmachers in einem Verfahren gemäß Anspruch 13 oder 14.

Weitere Einzelheiten und Vorteile der Erfindung werden anhand eines in der Zeichnung dargestellten Ausführungsbeispiels näher erläutert. Es zeigen:
- Figur 1:: eine schematische Ansicht eines Herzschrittmachers einer erfindungsgemäßen Anordnung und
- Figur 2:: eine schematische Ansicht des Zeitpunkts der Kardioversion im EKG.

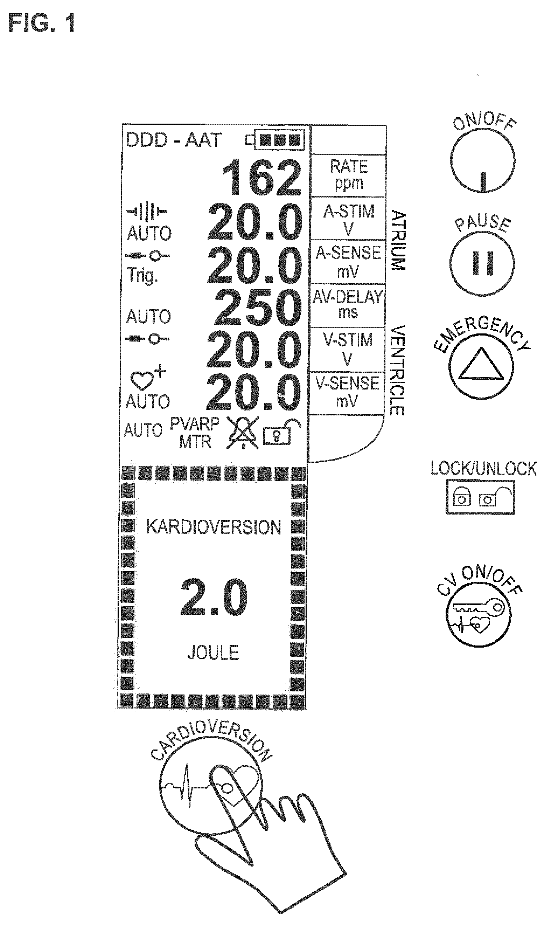

Figur 1 zeigt in einer schematischen Darstellung einen Herzschrittmacher einer erfindungsgemäßen Anordnung. Es handelt sich um einen externen, d.h. nicht implantierten Herzschrittmacher, der über ein oder mehrere Drähte mit dem Herzen eines Patienten verbindbar bzw. verbunden ist.

Der externe Herzschrittmacher ist in einem normalen Betriebsmodus betreibbar, in dem er zu bestimmten Zeitpunkten oder in Abhängigkeit der Herztätigkeit Stimulationsimpulse abgibt.

Der externe Herzschrittmacher ist darüber hinaus in einem Kardioversionsmodus betreibbar, der durch Betätigung einer Taste aktiviert wird, wie dies unten näher beschrieben wird. In dem Kardioversionsmodus lädt der Herzschrittmacher eine oder mehrere Kondensatoren, die mit dem Herzen verbundene bzw. verbindbare Elektroden mit Strom versorgen. Die Elektroden stehen mit dem Herzschrittmacher über ein oder mehrere Drähte zur Abgabe eines Kardioversionsschocks in Verbindung.

Durch Betätigung der Taste (CV on/off) wird der Kardioversionsmodus aktiviert. Sodann erscheint die Energieanzeige.

Der Nutzer hat die Möglichkeit, beispielsweise mittels eines Drehknopfes oder mittels eines sonstigen Elementes die Energie auszuwählen, die von der oder den Elektroden an das Herz abgegeben werden. In dem hier dargestellten Ausführungsbeispiel beträgt diese Energie 2,0 J.

Durch das Drücken der Taste angelangt man in das Kardioversionsmenü. Wie ausgeführt, wird nun auf dem unteren Bildschirm bzw. auf dem unteren Bereich des Bildschirms die eingestellte Energie angezeigt.

Wird erneut die Taste betätigt, wird der Kardioversionsmodus beendet. Dieser wird auch dann beendet, wird nach der Betätigung der Taste beispielsweise länger als 60 Sekunden oder länger als eine sonstige Zeitspanne keine Taste betätigt wird. In beiden Fällen kehrt der Herzschrittmacher in den normalen Betriebsmodus zurück.

Wird im Kardioversionsmodus die Schocktaste betätigt, wird der Kondensator geladen und gleichzeitig der Herzschrittmacher in den VVT-Modus gebracht.

Wird diese Taste vor dem Beenden der Kardioversion bzw. vor Ablauf einer bestimmten Zeitspanne (z.B. 15 Sekunden) losgelassen, bricht das Gerät den Kardioversionsmodus ab. Der Herzschrittmacher geht wieder über in vom Bediener eingestellten Herzschrittmachermodus. Das Gerät selbst bleibt im Kardioversionsmenü, in dem die abgegebene Energie angezeigt bzw. verändert werden kann.

Denkbar ist es, dass im Bereich der Kardioversionsanzeige ein Fortschrittsbalken für das Laden des Kondensators angezeigt wird. Beispielsweise ist nach etwa 15 Sekunden der Ladevorgang abgeschlossen und der Fortschrittsbalken füllt dann die Breite des Displays aus.

Das Gerät wartet nun beispielsweise drei Sekunden lang auf die nächst R-Zacke und triggert dann mit den zweiten Mitteln den Kardioversionsschock. Die vergangene Zeit wird durch einen Fortschrittsbalken angezeigt.

Nach beispielsweise drei Sekunden füllt der Fortschrittsbalken die Breite des Displays. Falls bis dahin keine R-Zacke erkannt wurde, wird der Kardioversionsvorgang automatisch abgebrochen und es erscheint eine entsprechende Fehlermeldung.

Konnte das Gerät dagegen den Schock erfolgreich abgeben, wird ein akustisches Signal ausgegeben und am Display wird eine Meldung, z. B. "Kardioversionsschock abgegeben" angezeigt. Diese Anzeige kann über eine bestimmte Zeitspanne, beispielsweise 20 Sekunden, verbleiben. Wird eine Taste betätigt, kann diese Anzeige früher wieder verschwinden.

War die Kardioversion nicht erfolgreich, kann beispielsweise eine höhere Schockenergie eingestellt werden und die Kardioversion kann wiederholt werden.

Wesentlich ist, dass nach dem Drücken der Taste d. h. nach dem Wechsel des Herzschrittmachers von dem normalen Herzschrittmacher-Modus in den VVT-Modus der Nutzer an einem Monitor beispielsweise an dem EKG-Monitor erkennen kann, ob der Herzschrittmacher korrekt arbeitet. Der Anwender kann die Pulsabgabe und damit auch die R-Zackenerkennung z.B. durch die Verwendung eines EKG-Monitors mit Schrittmachererkennung überprüfen, da diese Geräte die abgegebene Stimulationsimpulse im Oberflächen-EKG erkennen und auf dem Monitor markieren können.

Stellt der Nutzer bei visueller Überprüfung des Bildschirms fest, dass die Abgabe der Stimulationsimpulse durch die ersten Mittel nicht oder nicht korrekt erfolgt ist, beispielsweise weil kein Stimulationsimpuls erfolgte oder weil der Stimulationsimpuls in der vulnerablen Phase erfolgte, kann er die Taste loslassen, woraufhin das Gerät wieder in den normalen Herzschrittmacher-Modus wechselt und kein Kardioversionsschock abgegeben wird.

## Patentansprüche

1. Anordnung mit einem externen Herzschrittmacher sowie mit einer Ausgabeeinrichtung, wobei der externe Herzschrittmacher erste Mittel zur Erzeugung von Impulsen zur regelmäßigen Stimulation des Herzmuskels im VVT-Modus und zweite Mittel zur Erzeugung eines Stromstoßes bzw. Kardioversionsschocks zum Zwecke der Kardioversion aufweist, wobei ferner Aufzeichnungsmittel zur Ermittlung der Herzspannungskurve oder eines oder mehrerer Abschnitte der Herzspannungskurve vorgesehen sind sowie dritte Mittel, die mit der Ausgabeeinrichtung in Verbindung stehen und die so ausgebildet sind, dass sie der Ausgabeeinrichtung ein oder mehrere Signale zuführen, die sowohl die Herzspannungskurve bzw. deren wenigstens einen Abschnitt sowie im VVT-Modus seitens des Herzschrittmachers erzeugte Impulse umfassen, **dadurch gekennzeichnet, dass** die Anordnung Auswertemittel aufweist, die den zeitlichen Zusammenhang zwischen der R-Zacke der Herzspannungskurve und den im VVT-Modus seitens des Herzschrittmachers erzeugten Impulsen ermitteln.

2. Anordnung nach Anspruch 1, **dadurch gekennzeichnet, dass** Warnmittel vorgesehen sind, die mit den Auswertemitteln in Verbindung stehen, wobei die Warnmittel derart ausgebildet sein können, dass sie ein Warnsignal abgeben, wenn der genannte zeitliche Zusammenhang zwischen der R-Zacke der Herzspannungskurve und dem im VVT-Modus seitens des Herzschrittmachers erzeugten Impuls fehlerhaft ist.

3. Anordnung nach einem der vorhergehenden Ansprüche, **dadurch gekennzeichnet, dass** Sperrmittel vorgesehen sind, die mit den genannten Auswertemitteln in Verbindung stehen, wobei diese Sperrmittel derart ausgebildet sind, dass sie die Funktion der zweiten Mittel blockieren, wenn der genannte zeitliche Zusammenhang zwischen der R-Zacke der Herzspannungskurve und dem im VVT-Modus seitens des Herzschrittmachers erzeugten Impuls fehlerhaft ist.

4. Anordnung nach einem der vorhergehenden Ansprüche, **dadurch gekennzeichnet, dass** die Anordnung ein Betätigungselement aufweist, das derart ausgebildet ist, dass es bei seiner Betätigung das erste Mittel zur Erzeugung von Impulsen zur regelmäßigen Stimulation des Herzmuskels im VVT-Modus aktiviert.

5. Anordnung nach einem der vorhergehenden Ansprüche, **dadurch gekennzeichnet, dass** die Anordnung ein Betätigungselement aufweist, das derart ausgebildet ist, dass es bei seiner Betätigung das Aufladen eines Kondensators vornimmt.

6. Anordnung nach einem der vorhergehenden Ansprüche, **dadurch gekennzeichnet, dass** die Anordnung ein Betätigungselement aufweist, das so ausgebildet ist, dass es bei seiner Betätigung über eine bestimmte Zeitspanne hinweg die zweiten Mittel zur Erzeugung des Stromstoßes zum Zwecke der Kardioversion aktiviert.

7. Anordnung nach einem der vorhergehenden Ansprüche, **dadurch gekennzeichnet, dass** die Anordnung ein Betätigungselement aufweist, das so ausgebildet ist, dass es nach dem Loslassen vor Ablauf einer bestimmten Zeitspanne derart auf die zweiten Mittel einwirkt, dass kein Stromstoß zum Zwecke der Kardioversion abgegeben wird.

8. Anordnung nach einem der Ansprüche 4 bis 7, **dadurch gekennzeichnet, dass** es sich bei den vorgenannten Betätigungselementen um ein- und dasselbe Betätigungselement handelt.

9. Anordnung nach einem der vorhergehenden Ansprüche, **dadurch gekennzeichnet, dass** die Ausgabeeinrichtung Bestandteil des Herzschrittmachers ist oder dass die Anordnung einen EKG-Monitor umfasst und dass die Ausgabeeinrichtung Bestandteil des EKG-Monitors ist.

10. Anordnung nach einem der vorhergehenden Ansprüche, **dadurch gekennzeichnet, dass** die Ausgabeeinrichtung eine Anzeigeeinrichtung darstellt oder umfasst, wobei die Anzeigeeinrichtung vorzugsweise derart ausgebildet ist, dass die angezeigte Information durch einen Nutzer optisch und/oder akustisch wahrnehmbar ist.

11. Anordnung nach einem der vorhergehenden Ansprüche, **dadurch gekennzeichnet, dass** die Ausgabeeinrichtung ein Signal bereitstellt, das derart ausgeführt ist, dass basierend auf dem Signal ein Kardioversionsschock ausgelöst wird oder dessen Auslösung unterbunden wird.

12. Verfahren zur Überprüfung der Funktionsfähigkeit eines Herzschrittmachers, wobei der Herzschrittmacher in einem normalen Betriebsmodus betreibbar ist, in dem er Stimulationsimpulse abgibt, und in einem VVT-Modus betreibbar ist, in dem er einen Stromstoß bzw. Kardioversionsschock zum Zwecke der Kardioversion abgibt, wobei das Verfahren die Ermittlung der Herzspannungskurve und die Ermittlung von seitens des Herzschrittmachers im VVT-Modus abgegebenen Stimulationsimpulsen und die Prüfung des zeitlichen Zusammenhangs zwischen dem Vorhandensein einer R-Zacke der Herzspannungskurve und einem im VVT-Modus abgegebenen Stimulationsimpuls sowie die Ausgabe eines auf dieser Prüfung basierenden Signals umfasst.

13. Verfahren nach Anspruch 12, **dadurch gekennzeichnet, dass** die Ausgabe des Signals mittels eines EKG-Monitors erfolgt, der über eine Schrittmachererkennung verfügt.

14. Verwendung eines externen Herzschrittmachers in einem Verfahren gemäß Anspruch 12 oder 13.

## Claims

1. An arrangement having an external cardiac pacemaker and having an output device, wherein the external cardiac pacemaker has first means for generating impulses for a regular stimulation of the myocardium in the VV mode and second means for generating a current pulse or a cardioversion shock for the purpose of cardioversion, wherein furthermore recording means are provided for determining the electrocardiogram or one or more portions of the electrocardiogram, as well as third means which are connected to the output device and which are configured such that they supply one or more signals to the output device, said signal or signals comprising both the electrocardiogram or its at least one portion and, in the VVT mode, pulses generated by the cardiac pacemaker, **characterised in that** the arrangement has evaluation means which determine the time relationship between the R wave of the electrocardiogram and the pulses generated by the cardiac pacemaker in the VVT mode.

2. An arrangement in accordance with claim 1, **characterised in that** alarm means are provided which are connected to the evaluation means, with the alarm means being able to be configured such that they emit a warning signal when the named time relationship between the R wave, the electrocardiogram and the pulse generated by the cardiac pacemaker in the VVT mode is incorrect.

3. An arrangement in accordance with one of the preceding claims, **characterised in that** blocking means are provided which are connected to the named evaluation means, with these blocking means being configured such that they block the function of the second means when the named time relationship between the R wave of the electrocardiogram and the pulse generated by the cardiac pacemaker in the VVT mode is incorrect.

4. An arrangement in accordance with one of the preceding claims, **characterised in that** the arrangement has an actuation element which is configured such that, on its actuation, it activates the first means to generate pulses for the regular stimulation of the myocardium in the VVT mode.

5. An arrangement in accordance with one of the preceding claims, **characterised in that** the arrangement has an actuation element which is configured such that, on its actuation, it carries out the charging of a capacitor.

6. An arrangement in accordance with one of the preceding claims, **characterised in that** the arrangement has an actuation element which is configured such that, on its actuation over a specific period of time, it activates the second means to generate the current pulse for the purpose of cardioversion.

7. An arrangement in accordance with one of the preceding claims, **characterised in that** the arrangement has an actuation element which is configured such that, after being released before the end of a specific period of time, it acts on the second means such that no current pulse is emitted for the purpose of cardioversion.

8. An arrangement in accordance with one of the claims 4 to 7, **characterised in that** the aforesaid actuation elements are one and the same actuation element.

9. An arrangement in accordance with one of the preceding claims, **characterised in that** the output device is a component of the cardiac pacemaker; or **in that** the arrangement comprises an ECG monitor; and **in that** the output device is a component of the ECG monitor.

10. An arrangement in accordance with one of the preceding claims, **characterised in that** the output device represents or comprises a display device, with the display device preferably being configured such that the displayed information can be perceived visually and/or aurally by a user.

11. An arrangement in accordance with one of the preceding claims, **characterised in that** the output device provides a signal which is designed such that a cardioversion shock is triggered or its triggering is suppressed on the basis of the signal.

12. A method for checking the functional capability of a cardiac pacemaker, wherein the cardiac pacemaker can be operated in a normal operating mode in which it emits stimulation pulses and can be operated in a VVT mode in which it emits a current pulse or cardioversion shock for the purpose of cardioversion, wherein the method comprises determining the electrocardiogram and determining stimulation pulses emitted by the cardiac pacemaker in the VVT mode and checking the time relationship between the presence of an R wave of the electrocardiogram and a stimulation pulse emitted in the VVT mode as well as outputting a signal based on this check.

13. A method in accordance with claim 12, **characterised in that** the output of the signal takes place by means of an ECG monitor which has pacemaker recognition.

14. Use of an external cardiac pacemaker in a method in accordance with claim 12 or claim 13.

## Revendications

1. Dispositif doté d'un stimulateur cardiaque externe ainsi que d'un dispositif de sortie, le stimulateur cardiaque externe comportant des premiers moyens destinés à générer des impulsions de stimulation régulière du myocarde en mode VVT et des deuxièmes moyens destinés à générer un choc électrique ou choc de cardioversion à des fins de cardioversion, des moyens d'enregistrement étant en outre prévus pour déterminer l'électrocardiogramme ou une ou plusieurs portions de l'électrocardiogramme, ainsi que des troisièmes moyens, qui sont en liaison avec le dispositif de sortie et qui sont conçus de telle manière qu'ils envoient un ou plusieurs signaux au dispositif de sortie, lesquels signaux comprennent aussi bien l'électrocardiogramme ou l'au moins une portion de celui-ci que des impulsions générées par le stimulateur cardiaque en mode VVT, **caractérisé en ce que** le dispositif comporte des moyens d'évaluation, qui déterminent le rapport temporel entre l'onde R de l'électrocardiogramme et les impulsions générées par le stimulateur cardiaque en mode VVT.

2. Dispositif selon la revendication 1, **caractérisé en ce que** des moyens d'avertissement sont prévus, qui sont en liaison avec les moyens d'évaluation, les moyens d'avertissement pouvant être conçus de telle manière qu'ils émettent un signal d'avertissement, quand ledit rapport temporel entre l'onde R de l'électrocardiogramme et l'impulsion générée par le stimulateur cardiaque en mode VVT est incorrect.

3. Dispositif selon l'une des revendications précédentes, **caractérisé en ce que** des moyens de blocage sont prévus, qui sont en liaison avec lesdits moyens d'évaluation, ces moyens de blocage étant conçus de telle manière qu'ils bloquent le fonctionnement des deuxièmes moyens quand ledit rapport temporel entre l'onde R de l'électrocardiogramme et l'impulsion générée par le stimulateur cardiaque en mode VVT est incorrect.

4. Dispositif selon l'une des revendications précédentes, **caractérisé en ce que** le dispositif comporte un élément d'actionnement, qui est conçu de telle manière que, lors de son actionnement, il active le premier moyen destiné à générer des impulsions de stimulation régulière du myocarde en mode VVT.

5. Dispositif selon l'une des revendications précédentes, **caractérisé en ce que** le dispositif comporte un élément d'actionnement, qui est conçu de telle manière que, lors de son actionnement, il effectue la charge d'un condensateur.

6. Dispositif selon l'une des revendications précédentes, **caractérisé en ce que** le dispositif comporte un élément d'actionnement, qui est conçu de telle manière que, lors de son actionnement, il active les deuxièmes moyens destinés à générer le choc électrique à des fins de cardioversion durant une période déterminée.

7. Dispositif selon l'une des revendications précédentes, **caractérisé en ce que** le dispositif comporte un élément d'actionnement, qui est conçu de telle manière qu'il agit, après le relâchement avant l'écoulement d'une période déterminée, de telle manière sur les deuxièmes moyens qu'aucun choc électrique n'est émis à des fins de cardioversion.

8. Dispositif selon l'une des revendications 4 à 7, **caractérisé en ce que** les éléments d'actionnement mentionnés ci-dessus sont un même et unique élément d'actionnement.

9. Dispositif selon l'une des revendications précédentes, **caractérisé en ce que** le dispositif de sortie fait partie du stimulateur cardiaque ou **en ce que** le dispositif comprend un moniteur ECG et **en ce que** le dispositif de sortie fait partie du moniteur ECG.

10. Dispositif selon l'une des revendications précédentes, **caractérisé en ce que** le dispositif de sortie représente ou comprend un dispositif de signalisation, le dispositif de signalisation étant conçu de préférence de telle manière que l'information signalée peut être perçue de manière optique et/ou acoustique par un utilisateur.

11. Dispositif selon l'une des revendications précédentes, **caractérisé en ce que** le dispositif de sortie met à disposition un signal, qui est réalisé de telle manière qu'un choc de cardioversion est déclenché ou que son déclenchement est empêché sur la base du signal.

12. Procédé de vérification du bon fonctionnement d'un stimulateur cardiaque, le stimulateur cardiaque pouvant fonctionner dans un mode de fonctionnement normal, dans lequel il émet des impulsions de stimulation, et pouvant fonctionner dans un mode VVT, dans lequel il émet un choc électrique ou choc de cardioversion à des fins de cardioversion, le procédé comprenant la détermination de l'électrocardiogramme et la détermination d'impulsions de stimulation émises par le stimulateur cardiaque en mode VVT et le contrôle du rapport temporel entre la présence d'une onde R de l'électrocardiogramme et une impulsion de stimulation émise en mode VVT ainsi que la sortie d'un signal sur la base de ce contrôle.

13. Procédé selon la revendication 12, **caractérisé en ce que** la sortie du signal s'effectue au moyen d'un moniteur ECG, qui dispose d'une détection de stimulateur cardiaque.

14. Utilisation d'un stimulateur cardiaque externe dans un procédé selon la revendication 12 ou 13.
